Europäisches Patentamt

European Patent Office

Office européen des brevets

(11) Veröffentlichungsnummer: **0 027 664**

**A1**

## EUROPÄISCHE PATENTANMELDUNG

(21) Anmeldenummer: **80106520.2**

(22) Anmeldetag: **30.09.78**

(51) Int. Cl.³: **A 61 K 39/00**
A 61 K 39/395, A 61 K 37/02
C 07 G 7/00

(30) Priorität: **11.10.77 DE 2745680**

(43) Veröffentlichungstag der Anmeldung:
**29.04.81 Patentblatt 81/17**

(84) Benannte Vertragsstaaten:
**DE FR GB**

(60) Publication number of the earlier application in accordance with Art. 76 EPC: **0 001 443**

(71) Anmelder: **BEHRINGWERKE AKTIENGESELLSCHAFT**
**Postfach 1140**
**D-3550 Marburg/Lahn(DE)**

(72) Erfinder: **Bohn, Hans, Dr.**
**Oberer Eichweg 26**
**D-3550 Marburg/Lahn(DE)**

(72) Erfinder: **Weinmann, Ernst, Dr.**
**Sonnenweg 19**
**D-3550 Marburg/Lahn(DE)**

(74) Vertreter: **Meyer-Dulheuer, Karl-Hermann, Dr. et al,**
**HOECHST Aktiengesellschaft Zentrale Patentabteilung**
**Postfach 80 03 20**
**D-6230 Frankfurt/Main 80(DE)**

(54) Verwendung des Proteins PP5, seiner Derivate oder der gegen dieses Protein gerichteten Antikörper zur Konzeptionsverhütung oder Schwangerschaftsunterbrechung.

(57) Verwendung des Proteins PP₅, seiner Derivate oder der genen dieses Protein gerichteten Antikörper zur Konzeptionsverhütung oder Schwangerschaftsunterbrechung.

EP 0 027 664 A1

- 1 -

BEHRINGWERKE AKTIENGESELLSCHAFT     HOE 77/B 023   Dr.LI/Pa

Verwendung des Proteins PP$_5$, seiner Derivate oder der
gegen dieses Protein gerichteten Antikörper zur Konzeptionsverhütung oder Schwangerschaftsunterbrechung

Gegenstand der Erfindung sind Mittel zur Konzeptionsverhütung und zur Schwangerschaftsunterbrechung bei
Primaten auf immunologischer Basis und Verfahren zu ihrer
Herstellung.

Kontrazeptive Mittel, welche auf der Grundlage immunologischer Reaktionen wirksam sind, wurden mehrfach beschrieben. So ist schon versucht worden, durch Antiseren gegen
Extrakte tierischer Plazenten bei Kaninchen zu verhüten konzeption
und bei Affen und Mäusen Fehlgeburten herbeizuführen. In
dem schwangerschaftsspezifischen $\beta_1$-Glykoprotein wurde
ein wirksames Mittel zur Konzeptionsverhütung und zur
Schwangerschaftsunterbrechung bei Primaten gefunden (vgl.
DOS 23 45 953). Ein Antigen zur immunologischen Fruchtbarkeitskonzrolle, bestehend aus proteinartigen Fortpflanzungshormonen, ist in DOS 26 46 023 beschrieben, ein
kontrazeptiver Impfstoff in DOS 26 18 546 mit einem ähnlichen Aufbau. Insbesondere bei den letztgenannten wird
als Mangel angesehen, daß Antihormone eine über die
Fortpflanzungsfähigkeit hinausgehende Wirksamkeit besitzen, die nicht erwünscht ist. Auch die auf der Basis
der DOS 23 45 953 aufgebauten Mittel haben nicht immer die
immunologische Wirksamkeit gezeigt, wie sie für eine erfolgreiche Konzeptionsregulierung erforderlich erscheint.
Es hat deshalb nicht an Versuchen gefehlt, die immunogene
Wirksamkeit der Antigene zu verbessern (vgl. DOS 25 43 994).
Auch die DOS 26 46 223 und 25 18 546 bedienen sich modifizierter Hormonantigene.

Neben den von H.Bohn in Arch. Gynäk., 212, S. 165-175
(1972) beschriebenen löslichen Antigenen der menschlichen
Plazenta sind als Inhaltsstoffe dieses Organs die Proteohormone Choriongonaditropin, Plazentalactogen und Chorion-

-2-

thyrotropin beschrieben, ferner die Enzyme Cystinaminopeptidase, hitzestabile alkalische Phosphatase und 17-ß-
Hydroxisteroid-Dehydrogenase. Über die Eignung des
schwangerschaftsspezifischen $\beta_1$-Glykoproteins als Bestandteil eines kontrazeptiven Impfstoffes wurde bereits eingangs berichtet. Ebenso über die Verwendung der betreffenden Hormone in diesem Sinne.

Es wurden nun überraschend gefunden, daß immunologisch zur
Antikörperinduktion gegen das plazentenspezifische Glycoprotein $PP_5$ befähigte Substanzen hervorragende immunologische Antikonzeptiva darstellen. Das $PP_5$ ist ein bereits
beschriebenes Placentaprotein. Es ist gemäß einem in der
DOS 26 16 984 beschriebenen Verfahren erhältlich. Es zeigt
eine elektrophoretische Wanderung in Agargel im Bereich
der $\beta_1$-Globuline. In einem Polyacrylamidgel wandert es
im Vergleich zu Albumin = 100 mit einer relativem Beweglichkeit von 75. Aufgrund seines Verhaltens bei der
Gelfiltration auf quervernetztem Dextran wurde ein Molekulargewicht für $PP_5$ von etwa 50.000 abgeleitet. Am einfachsten wird es durch seine immunologische Reaktion mit
einem spezifischen Anti-$PP_5$-Serum charakterisiert.

Gegenstand der Erfindung ist demnach ein Mittel zur Konzeptionsverhütung und zur schwangerschaftsunterbrechung
und Herbeiführung von Fehlgeburten bei Primaten, insbesondere Menschen, dadurch gekennzeichnet, daß es als
wesentlichen Bestandteil eine wirksame Menge einer Substanz enthält, welche bei parenteraler Verabreichung mit
dem Protein $PP_5$ immunologisch zu reagieren oder immunologisch reaktive Substanzen, Antikörper, die gegen das
$PP_5$ gerichtet sind, zu induzieren vermag. Zunächst sind
hierzu zu rechnen Antikörper mit immunologischen Determinaten, die gegen das $PP_5$ gerichtet sind, vorzugsweise
das Anti-$PP_5$ selbst, aber auch Antikörper, welche mit
Derivaten des $PP_5$ erhalten worden sind. Von den Substanzen, welche Antikörper gegen das $PP_5$ zu induzieren vermögen, sind an erster Stelle das $PP_5$ zu nennen, ferner

die davon abgeleiteten Derivate. Von den Derivaten des $PP_5$ haben insbesondere solche Bedeutung, welche durch einfache Umsetzung des $PP_5$ mit einer Proteine modifizierenden Verbindung herstellbar sind sowie die $PP_5$-Konjugate mit Haptenen.

Bei derartigen Umsetzungen ist von zwei Grundprinzipien auszugehen. Sie führen beide zur Veränderung der nativen Struktur und damit zu einer Denaturierung des Proteins:

1. Verfahren zur chemischen Veränderung des $PP_5$ mit Hilfe von Reaganzien, welche zu einer Veränderung vorhandener chemischer Gruppen führen.

2. Verfahren, die zu einer chemischen Veränderung des $PP_5$ durch Einführung neuer Gruppen in das Molekül bzw. die zur Verbindung des Moleküls mit niedrig- oder hochmolekularen Verbindungen führen.

Zu den genannten Verfahren sind diejenigen zu zählen, die in Proteinen eine oder mehrere der folgenden Gruppen zu verändern vermögen: Amino, Guanidyl, Imidazol, Indol, aliphatisches Hydroxyl, Amid, Thioäther, Disulfid, Sulhydryl, Phenol und Carboxyl. Hierfür ist eine Reihe von Reaktionen aus der Literatur bekannt, zu denen hauptsächlich die nachfolgend genannten Reagenzien unter den beispielhaft erwähnten Bedingungen einzusetzen sind.

1.1 Oxydation mit Jodosobenzoat, Porphyrindin, Ferrocyanid oder Jod, wobei in der Regel eine Konzentration des Oxydationsmittels von 0,001 - 0,01 M, ein pH-Wert von 7, eine Temperatur von 0 - 25$^{\circ}$C und eine Umsetzungsdauer von 5 - 30 Minuten anzuwenden sind. Die Oxydation mit Jod wird in Gegenwart von Jodid in hoher Konzentration bei einem pH-Wert von 1 - 7 ausgeführt. Die Oxydation kann auch mit Wasserstoffperoxyd vorgenommen werden. Die bevorzugten Bedingungen sind dann:

Konzentration des Oxydationsmittels etwa 0,005 M, pH-Wert etwa 6,6, Temperatur etwa $25^{\circ}$ und Reaktionsdauer 0,5 - 40 Stunden.

1.2 Reduktion mit Cystein, Thioglykolsäure, Thioglykol, Cyanid oder Sulfid, vorzugsweise unter folgenden Bedingungen: Konzentration des Reduktionsmittels 0,001 bis 0,1 M, pH-Wert 7 - 8, Temperatur etwa $25^{\circ}$C und Reaktionsdauer 0,1 - 4 Stunden.

Die Reaktionsbedingungen können im einzelnen der Fachliteratur entnommen werden. Sie sind beispielsweise referiert von H.S. Olcott und H. Fraenkel-Conrat, Chem. Rev. 41, S. 151 ff (1974). Zudem ist ein Teil der Reagenzien und der Verfahrensbedingungen aus H.E. Schultze und J.F. Heremans, Molecular Biology of Human Proteins (1966), S. 40 - 41 und den darin auf S. 55 ff. angegebenen Referenzen zu ersehen.

Mit der Einführung neuer Gruppen in das $PP_5$ werden Derivate hergestellt, die vom immunologischen Standpunkt aus in der Regel als Hapten-Verbindungen bezeichnet werden. Nach einer anerkannten Definition ist ein "Hapten" eine proteinfreie Substanz, die mit einem spezifischen gegen seine Konfiguration gerichteten Antikörper reagieren kann, die ihrerseits jedoch nicht zur Bildung nachweisbarer Antikörpermengen befähigt ist. Ein Hapten bewirkt eine Immunantwort nur in Verbindung mit einem Trägerprotein. Die meisten Haptene sind niedermolekulare Verbindungen mit einem Molekulargewicht $<1000$. Es werden jedoch auch Makromoleküle wie beispielsweise Pneumokokkenpolysaccharide als Haptene angesehen. Eine Hapten-Protein-Verbindung führt in der Regel Induktion von zwei Typen von Antikörpern, wobei Spezifitäten gegen die Haptengruppierung und gegen den Proteinträger entstehen. Im Falle des $PP_5$ gilt, daß dessen Hapten-Verbindungen auch gegen das Trägerprotein im homologen System Antikörper induzieren.

Als Haptene im Sinne der Erfindung kommen die in der Literatur als solche beschriebenen chemischen Gruppen und Verbindungen in Frage. Insbesondere gehören hierzu aromatische Ringsysteme, Steroide, Peptide, Purine, Pyrimidine, Penicillin und dessen Derivate, aber auch Einzelmoleküle wie beispielsweise Jod, und entsprechend einer hier vorgenommenen erweiterten Definition der an den Träger von $PP_5$ zu bindenden Substanzen auch hochmolekulare Körper mit Peptid- bzw. Protein- und Kohlenhydratcharakter, die ihrerseits selbst antigene Eigenschaften haben.

Die Verfahren zur Herstellung der Derivate des $PP_5$ nach dem genannten Grundprinzip (2.) unter Verwendung von Haptenen sind als allgemein anwendbare Reaktionen für die Einführung von Proteine modifizierenden chemischen Gruppen und von Haptenen in Eiweißkörpern unter Ausbildung kovalenter Verknüpfung zwischen chemischen Substanzen und Proteinen bekannt. Insbesondere handelt es sich dabei um Reaktionen, die für die Modifikation einzelner Gruppen in Proteinmolekülen beschrieben sind. Auch diese Reaktionen sind in den Arbeiten von Olcott und Fraenkel-Conrat und von Schultze-Heremans beschrieben. Beispiele solcher Verfahren sind:

2.1 Die Alkylierung mit

Jodacetat, Jodacetamid, (0,05 - 0,1 M, pH 7 - 8, 0 - 25°C, 0,5 - 2 Stunden) oder

Dinitrofluorobenzol: (0,17 M, pH 7-8, 25°, 2 Stunden).

2.2 Die Acylierung mit

| | |
|---|---|
| Keten | (pH 5-8, 0-25°, 5-30 Minuten), |
| Essigsäureanhydrid | (pH 7-8, 0°, 30 Minuten), |
| Kohlenstoffsuboxid | (pH 5-8, 0-25°, 5-30 Minuten), |
| Aziden, Benzoyl-, Carbobenzoxy- oder | |
| Benzolsulfonylchlorid | (pH 7-9, 0-25°, 0,5-2 Stunden), |
| Salpetriger Säure | (1M, pH 4, 30 Minuten) |
| Jod | (pH 5-11, -5°-25°, 0,5-3 Stunden im Gegensatz zu oben be- |

schriebener Oxydation mit geringerer Jodmenge und niedrigerer (Jodidkonzentration),

Formaldehyd (1-2M, 25$^{\circ}$, bei pH 7-8, 1 Stunde, bei pH 11, 1-4 Tage),

Epoxiden (1-2M, pH 5-6, 10 Minuten),

Senfgas (pH 5-6, 25$^{\circ}$, 0,5-4 Stunden),

Säure/Alkohol (Mineralsäure in absolutem Alkohol) (0,01-0,1M, 0-25$^{\circ}$, 1-2 Tage),

Methyldiazoacetat, Diazoacetamid (pH 5 (Ester), pH 6 (Amid), 0$^{\circ}$),

p-Chloromercuribenzoat ($10^{-5}$-$10^{-2}$M, pH 7, 25$^{\circ}$, 5-30 Minuten),

Diazonium-Verbindungen (pH 7-9, 25$^{\circ}$, 30 Min.) oder

o-Methylisoharnstoff (0,5M, pH 10,5, 0$^{\circ}$, 3 Tage).

Für einige der obengenannten Gruppen ist nachfolgend auf die Verfahren näher eingegangen:

a) <u>Jodierung</u> (Literatur: Kabat and Mayer's Experimental Immunochemistry, sec. edition 1961, 816-818)

Jod wird als Jodanion mit Tyrosinresten des Proteins zur Umsetzung gebracht, wobei ein oder zwei Jodatome in das Tyrosinmolekül eingeführt werden und das Jodprotein entsteht. In gleicher Weise kann die Jod-Verbindung des $PP_5$ hergestellt werden.

b) <u>Diazotierung und Kupplung</u> (Literatur: Kabat and Mayer's Experimental Immunochemistry, sec. edition 1961, 798-799)

Hierzu wird eine Verbindung mit einer aromatischen Aminogruppe, beispielsweise die Arsanilsäure oder Sulfanilsäure, diazotiert und die Diazoniumverbindung sodann mit dem Protein umgesetzt. Dabei gehen vornehmlich Tyrosinreste, aber auch Histidin- und Lysinreste des Proteins eine kovalente Verbindung mit der aromatischen Komponente ein. Nach diesem Verfahren kann die Diazo-Arsanilsäure- oder die Diazobenzol-Sulfonsäure-Verbindung des $PP_5$ hergestellt werden.

c) **Umsetzung mit Vinylsulfonverbindungen** (in Analogie zur Reaktion von Reaktivfarbstoffen, die als Reaktivkomponente die Vinylsulfongruppe enthalten, mit Zellulose oder Wolle).

Aliphatische oder aromatische Vinylsulfonderivate und Schwefelsäurehalbester von ß-Hydroxyäthylsulfonen können mit den Aminogruppen und/oder Hydroxylgruppen des Proteins zur Reaktion gebracht werden. Auf diese Weise kann z.B. die Hapten-Verbindung des $PP_5$ mit 1-Aminobenzol-4ß-Hydroxyäthylsulfon-Schwefelsäure erhalten werden.

d) **Reaktion mit Isocyanat- und Isothiocyanat-Verbindungen** (Literatur: Kabat und Mayer's Experimental Immuno- chemistry, sec. edition 1961, 809-811).

Diese Reaktion, die über die freien Aminogruppen des Proteins abläuft, erlaubt in Analogie zu den bekannten Verfahren der Proteinchemie die Umsetzung von Haptenen mit der entsprechenden chemischen Gruppierung auch mit dem $PP_5$.

e) **Dinitrophenylierung** (Literatur: Carsten, M.E.; Eisen, H.N., J.Am.Chem.Soc. 77, 1273 (1955)).

Die in der Regel mit Dinitrobenzolsulfonat oder Dinitrofluorbenzol durchgeführte Reaktion führt zur Umsetzung der freien Aminogruppen des Proteins unter Bildung von Dinitrophenylderivaten. Auf diese Weise wird dinitrophenyliertes $PP_5$ erhalten.

f) **Reaktion mit gemischten Anhydriden** (Literatur: Kabat and Mayer's Experimental Immunochemistry, sec. edition 1961, 813-815).

Dieses Verfahren ist geeignet, um eine Reihe verschiedener Haptene, die zunächst durch Acylierung in ein gemischtes Anhydrid überführt werden müssen, mit Aminogruppen des Proteins zu verknüpfen. Zudem lassen sich durch direkte Umsetzung von Proteinen mit Anhydriden in bekannter Weise Säureamide der allgemeinen Formel R-CO-NH————Protein herstellen, wobei R ein beliebiges Hapten sein kann. Mit Hilfe dieser Reaktion läßt sich beispielsweise die $PP_5$-Hapten-Verbindung mit R=Benzyl als Hapten herstellen.

g) <u>Reaktion mit Carbodiimiden</u> (Literatur: Makino, T. et. al., Contraception 8 (2), 133 (1973)).

Die Carbodiimide der allgemeinen Formel R-N=C=N-R (R = ein beliebiger organischer Rest) sind geeignet, um Carboxyl-Gruppen enthaltende Haptene mit Aminogruppen von Proteinen zu verknüpfen. Auch hierbei wird als Reaktionsprodukt die Verbindung R-CO-NH-Protein erhalten, wobei R jedes beliebige Hapten, das eine Carboxylgruppen besitzt, sein kann. Mit Hilfe dieser Reaktion wird beispielsweise die Hapten-Verbindung des $PP_5$ mit Cyclohexancarbonsäure als Hapten erhalten.

Das immunologische Verhalten des $PP_5$ läßt sich erfindungsgemäß auch durch Verknüpfung des Glykoproteins mit einem Peptid oder Protein verändern. Die Verknüpfung kann entweder kovalent oder durch Komplexbildung vorgenommen werden. Als Reaktionspartner sind hier Peptide, beispielsweise auch solche mit eigener biologischer Funktion, verwendbar, z.B. Dekapeptide wie der LRF (Luteinisierendes Hormon Relasing Factor). Der LRF ist beispielsweise in der gleichen Weise mit dem $PP_5$ zu verknüpfen wie dies für dessen Verknüpfung mit Albumin von T. Makino u.a. in Contraception <u>8</u>, 2 (1973), S. 133 ff., beschrieben ist. Die zugrundeliegende Reaktion geht auf die Carbodiimid-Aktivierung der Carboxylgruppen mit Carbodiimid-Verbindungen zurück.

Eine weitere Möglichkeit zur Veränderung des immunologischen Verhaltens des $PP_5$ besteht in der Verknüpfung des Proteins mit einer zweiten Proteinsubstanz in der Weise, wie dies beispielsweise für die Verknüpfung von Enzymen mit Proteinen bekannt ist, wobei das $PP_5$ in der Reaktion einen der beiden Partner ersetzen kann. Für die Verbindung der beiden Proteine miteinander sind eine Reihe von Verfahren geläufig. Sie verwenden einzelne Gruppen von aktivierenden Substanzen wie beispielsweise Carbodiimide, Cyanurchlorid, p,p-Diflour-m,m-dinitrophenylsulfon oder Glutardialdehyd. Das $PP_5$ kann z.B. mit Hilfe von Glutardialdehyd in einer Reaktion analog der, die von S. Avrameas in Immunochemistry 6, (1969), 43 - 52, für die Kopplung von Gammaglobulin mit Peroxydase beschrieben ist, mit einer Reihe von Proteinen durchgeführt werden. Vorzugsweise wird im Sinne der vorliegenden Erfindung das als immunisierendes Agens vorgesehene $PP_5$ mit einer Proteinsubstanz verbunden, die selbst als Impfstoffkomponente wirksam ist, wie z.B. Tetanustoxoid. Andere geeignete Träger für das $PP_5$ sind beispielsweise Flagellin, Hämocyanin, Dextran, abgetötete Viren oder Bakterien, deren Toxoide sowie synthetische Polypeptide und Polynucleotide und schließlich biologisch abbaubare Polymere wie Polymilchsäure, Polyglykolsäure oder Kollagen.

Eine bevorzugte Ausführungsform hierfür stellt das Reaktionsprodukt von $PP_5$ mit Tetanustoxoid dar, welche darin kovalent in einem Molverhältnis von etwa 10:1 bis 1:1 z.B. mit Hilfe von Glutardialdehyd verbunden sind.

Es gehören zum Gegenstand der Erfindung Verfahren zur Herstellung von $PP_5$-Derivaten, d.h. proteinmodifizierten Verbindungen von $PP_5$, das durch Extraktion von menschlicher Plazenta erhalten worden und zur Proteinmodifizierung einem Verfahren zur Oxidation, Reduktion, Alkylierung oder Acylierung von Proteinen, zur Herstellung einer kovalenten Bindung zwischen Protein und Haptenen oder zwischen Proteinen untereinander unterworfen worden ist.

Die erhaltenen Reaktionsprodukte werden entsprechend bekannten Techniken in die für die parenterale Injektion geeignete Zubereitung überführt und wie bei Impfstoffen üblich ggf. mit stabilisierenden Zusätzen und anorganischen oder organischen Immunadjuvantien versehen, um auf diese Weise als aktiv immunisierendes Agens für die Geburtenkontrolle eingesetzt werden zu können.

Als stabilisierende Zusätze kommen Stoffe in Frage, welche die Haltbarkeit der Präparate verbessern, insbesondere niedrig-molekulare Kohlenhydrate oder Eiweißstoffe oder deren Derivate wie abgebautes und wiedervernetztes Kollagen, beispielsweise das unter dem Warenzeichen Haemaccel$^{(R)}$ erhältliche Gelatine-Produkt, ggf. mit weiteren Zusätzen wie Aminosäure-Salzen, z.B. Natriumglutaminat. Die Menge derartiger Zusätze beträgt zweckmäßig 0,5 % bis 5 %, zuweilen bis zu 10 %. Das immunisierende Agens kann danach flüssig oder in trockener, zweckmäßig in gefriergetrockneter Form, zur Verfügung gestellt werden. Letzteres wird vor der Applikation im Wasser oder einem physiologisch verträglichen Medium aufzulösen sein.

Für die wirksame Dosis und deren Verabreichung gelten zwei grundsätzlich verschiedene Überlegungen. Werden direkt mit $PP_5$ reagierende Antikörperpräparationen parenteral verabreicht, genügt häufig eine einmalige Injektion von etwa 50 bis 500 mg Antikörpereiweiß pro kg Körpergewicht. Bei den Präparationen, mit welchen eine Antikörperbildung gegen das $PP_5$ induziert werden soll, verwendet man mehrfach Einzeldosen von etwa 0,1 bis 50 mg. Diese wird zweckmäßigerweise 1 bis 5 Mal in Intervallen von 1 - 3 Wochen verabreicht.

Die folgende Versuchsbeschreibung zeigt den Erfolg der passiven sowie aktiven Immunisierung von Affen mit $PP_5$-Antikörpern bzw. Derivaten des $PP_5$.

1. Unterbrechung der Schwangerschaft durch passive Immunisierung

A. Gewinnung von Antiseren und Reinigung der Antikörper.

40 Kaninchen werden mit menschlichem $PP_5$ immunisiert.

Dazu wird das $PP_5$ oder dessen Derivat in physiologischer Kochsalzlösung gelöst (Konzentration 0,06 mg/3 ml) und unter Zusatz von Aluminiumhydroxid zu einer Suspension verrührt. Die Kaninchen erhalten an 5 aufeinanderfolgenden Tagen je 0,05 mg Protein in 3 ml Suspension pro Tier i.v. injiziert. Anschließend läßt man die Tiere 10 Tage ruhen.

Dann immunisiert man wieder an 5 aufeinanderfolgenden Tagen mit der oben angegebenen gleichen Menge Antigen, läßt die Tiere wieder 10 Tage ruhen und injiziert schließlich nochmals an 5 aufeinderfolgenden Tagen je 0,05 mg des Antigens. Nach einer erneuten Wartezeit von 7 bis 9 Tagen werden die Tiere entblutet. Nach dem Gerinnen des Blutes wird das Serum vom Blutkuchen abgeschleudert und gewonnen.

Aus dem Serum der Tiere werden die Antikörper durch Fällung mit $(NH_4)_2SO_4$ (30 % w/v) und durch Chromatographie mittels DEAE-Cellulose und 0,03 molarem Phosphatpuffer pH 7,0 isoliert. Die Antikörper wandern dabei ungehindert durch die Säule, die übrigen Serumproteine bleiben an die DEAE-Cellulose gebunden. Das Eluat mit den Antikörpern wird auf 5 % Eiweiß ankonzentriert, mit 2,25 % Glycin versetzt, sterilfiltriert, in Portionen zu 5 ml abgefüllt und lyophilisiert. Jede Abfüllung enthält 250 mg Eiweiß.

B Unterbrechung der Schwangerschaft

Trächtige Affen der Gattung Cynomolgus erhielten zwischen dem 21. und 27. Schwangerschaftstag an drei aufeinanderfolgenden Tagen je 250 mg des Antikörper-Präparates aus dem Kaninchenserum gegen $PP_5$ in 5 ml gelöst intravenös verabreicht. 5 von 7 dieser passiv immunisierten Tiere abortierten zwischen dem 33. und 48. Tag der Schwangerschaft. Ein·anderer Affe (Nr. 76) hatte eine Totgeburt am 128. Tag. Nur 1 Affe (Nr. 83) trug seine Schwangerschaft normal aus (vgl. Tabelle I).

Tabelle I

Passive Immunisierung mit $PP_5$

| Cynommolgus Affe | Applikation der $PP_5$-Antikörper Tag der Schwangerschaft | Abort Tag der Schwangerschaft | Geburt |
|---|---|---|---|
| 75 | 21 - 23 | 33 | nein |
| 76 | 25 - 27 | 128 | ja |
| 77 | 24 - 26 | 48 | nein |
| 78 | 24 - 26 | 38 | nein |
| 81 | 25 - 27 | 44 | nein |
| 82 | 23 - 25 | 35 | nein |
| 83 | 24 - 26 | - | ja |

2. Konzeptionsverhütung durch aktive Immunisierung mit Derivaten des $PP_5$

Geschlechtsreife weibliche Affen (Cynomolgen) wurden über einen Zeitraum von 6 Wochen mit insgesamt 3 mg eines Derivates von $PP_5$ in isotonischer Kochsalzlösung und fein verteiltem Aluminiumhydroxyd als Adjuvans abwechselnd intravenös und subkutan immunisiert. Nach der Immunisierung wurden die Tiere jeweils zum Zeitpunkt der Ovulation für 3 Tage mit einem befruchtungsfähigen männlichen Tier zur Paarung zusammengebracht und dann das Ergebnis der Kopulation registriert. Die gegen $PP_5$ immunisierten weiblichen Affen waren gegenüber nichtbehandelten Kontrolltieren in ihrer Fortpflanzungsfähigkeit stark gehemmt. Das Ergebnis bei den immunisierten Tieren war wie folgt:

Von den 11 Affen, welche mit $PP_5$-Präparationen immunisiert worden waren, wurden nur 2 nach der ersten Kopulation trächtig. Ein weiterer Affe konzipierte erst nach der 2. Kopulation, 3 nach der dritten und einer nach der 4. Kopulation. Von den insgesamt 7 trächtig gewordenen Affen haben bereits 2 wieder abortiert.

Die Kontrollgruppe bestand aus 5 Tieren, die in der gleichen Weise wie die übrigen Tiere gehalten wurden. Sie erhielten jedoch statt der $PP_5$-Präparation lediglich eine isotonische Kochsalzlösung mit fein verteiltem Aluminiumhydroxid. In dieser Gruppe wurden 3 Affen nach der 1. Kopulation trächtig, ein weiteres Tier nach der 2. und das letzte nach der 4. Kopulation. Alle Tiere brachten gesunde Junge zur Welt.

Die nachfolgenden Beispiele dienen zur weiteren Erläuterung der Erfindung.

Beispiel 1:

Modifizierung von $PP_5$ mit diazotierter Sulfanilsäure

a) Herstellung des Diazoniumsalzes

1 g Sulfanilsäure werden bei Zimmertemperatur in 100 ml 0,1 N HCl gelöst, dann in Eiswasser gestellt und unter Rühren auf $0^{\circ}C$ abgekühlt. Die Sulfanilsäure fällt dabei als Salz aus. In die Suspension läßt man dann 50 ml einer kalten 1 %igen Lösung von Natriumnitrit in Wasser langsam (im Verlauf von 1 Stunde) unter Rühren eintropfen; das Ende der Reaktion wird durch die Blaufärbung von Kaliumjodid-Stärkepapier angezeigt.

b) Herstellung der Hapten-Verbindung

Die Konjugation von $PP_5$ mit der diazotierten Sulfanilsäure erfolgt bei schwach alkalischem pH. Man löst dazu 20 mg $PP_5$ in 2 ml eines 0,2 M Natriumphosphatpuffers von pH 8,2 und kühlt die Lösung auf $4^{\circ}C$ ab. Dann gibt man 0,11 ml der Diazoniumsalzlösung hinzu und rührt 4 Stunden bei 4$^O$C. Der pH-Wert, der nicht unter 8,0 absinken soll, wird dabei durch Zugabe von 0,2 N NaOH korrigiert. Anschließend läßt man die Lösung 15 Stunden bei 4$^O$C stehen. Das modifizierte Protein wird dann mehrere Tage gründlich gegen Wasser dialysiert und schließlich lyophilisiert.

Eine Dosis des immunisierenden Agens hat folgende Zusammensetzung: 0,2 mg des Derivates von PP$_5$ gelöst in 3 ml isotonischer Kochsalzlösung enthaltend 0,05 % Aerosil, 5 % Haemaccel$^{(R)}$ und als Konservierungsmittel 0,15 mg Natriumtimerfonat.

Beispiel 2:

Modifizierung von PP$_5$ durch Kupplung mit Tetanustoxoid

---

Die kovalente Verknüpfung von PP$_5$ mit Tetanustoxoid wird in wäßriger Lösung bei pH 5,0 unter Verwendung eines wasserlöslichen Carbodiimids durchgeführt:

20 mg PP$_5$ und 60 mg Tetanustoxoid werden in 5,8 ml Wasser gelöst, durch Zugabe von 1/10 n Salzsäure auf pH 5,0 eingestellt und dann unter Rühren mit 6,6 mg N-Äthyl-N'(3-Dimethylaminopropyl) - Carbodiimid-HCl versetzt. Das Gemisch wird 3 Stunden bei Zimmertemperatur (20$^O$C) und anschließend 20 Stunden bei +4$^O$C gerührt, dann neutralisiert und gegen isotone Kochsalzlösung dialysiert.

Eine Dosis des immunisierenden Agens hat folgende Zusammensetzung:

0,5 mg Protein in 3 ml isotonischer Kochsalzlösung und 1,5 mg Al(OH)$_3$ oder ein anderes  immunologisches Adjuvans).

Patentanspruch:

Verwendung des Proteins PP$_5$, seiner Derivate oder der gegen dieses Protein gerichteten Antikörper zur Konzeptionsverhütung oder Schwangerschaftsunterbrechung.

Europäisches
Patentamt

**EUROPÄISCHER RECHERCHENBERICHT**

| | **EINSCHLÄGIGE DOKUMENTE** | | | **KLASSIFIKATION DER ANMELDUNG** (Int. Cl.³) |
|---|---|---|---|---|
| Kategorie | Kennzeichnung des Dokuments mit Angabe, soweit erforderlich, der maßgeblichen Teile | | betrifft Anspruch | |
| D,A | <u>DE - A1 - 2 345 953</u> (BEHRINGWERKE) <br> * Anspruch 1 * <br><br> -- | | 1 | A 61 K 39/00 <br> A 61 K 39/395 <br> A 61 K 37/02 <br> C 07 G 7/00 |
| P,A, D | <u>DE - A1 - 2 616 984</u> (BEHRINGWERKE) <br> * Anspruch 1 * <br><br> ---- | | 1 | |

**RECHERCHIERTE SACHGEBIETE** (Int. Cl.³)

A 61 K 37/02
A 61 K 39/00
A 61 K 39/395
C 07 G 7/00

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Berlin | 23-01-1981 | KNAACK |

EPA form 1503.1 08.78